# EUROPÄISCHE PATENTSCHRIFT

(11) **EP 2 566 631 B1**
(45) Veröffentlichungstag und Bekanntmachung des Hinweises auf die Patenterteilung: **02.03.2016**
(21) Anmeldenummer: 11729896.8
(22) Anmeldetag: 15.04.2011
(51) Int. Cl.: B05C 17/005, A61C 5/06, A61B 17/88

(54) **KARTUSCHENSYSTEM MIT VERBUNDENEN FÖRDERKOLBEN**
CARTRIDGE SYSTEM WITH JOINED PISTONS
SYSTÈME DE CARTOUCHE À PISTONS CONNEXÉS

(30) Priorität: 04.05.2010 DE 102010019220
(43) Veröffentlichungstag der Anmeldung: 13.03.2013
(73) Patentinhaber: Heraeus Medical GmbH, 61273 Wehrheim (DE)
(72) Erfinder: VOGT, Sebastian, 99092 Erfurt (DE); BUECHNER, Hubert, 90491 Nuernberg (DE)
(74) Vertreter: Panten, Kirsten
(86) Internationale Anmeldenummer: PCT/EP2011/001919
(87) Internationale Veröffentlichungsnummer: WO 2011/137971

(56) Entgegenhaltungen:
- EP-A1- 1 004 353
- WO-A1-2008/135714
- DE-A1- 2 521 392
- FR-A1- 2 702 396
- GB-A- 2 082 686

## Beschreibung

Die Erfindung betrifft ein Kartuschensystem zum Mischen und Applizieren eines Mischguts, insbesondere eines medizinischen Zements, umfassend zumindest zwei parallel zueinander angeordnete Kartuschen und eine Auslassöffnung, wobei die Kartuschen jeweils zumindest eine Öffnung umfassen, die die Kartuschen mit der Auslassöffnung verbinden, und die Kartuschen seitlich durch Kartuschenwände begrenzt sind und jeweils einen Förderkolben zum Austreiben von Ausgangskomponenten des Mischguts aus den Kartuschen durch die Öffnungen umfassen, und wobei zumindest zwei Förderkolben fest miteinander verbunden sind.

Kartuschensysteme zum Mischen und Applizieren eines Mischguts können aus mehreren Komponenten bestehen und sollen eine sichere Aufbewahrung und einen sicheren Verschluss für Komponenten in zumindest zwei Kartuschen vor ihrer Verwendung gewährleisten. Unmittelbar vor der Applikation des Mischguts soll das Kartuschensystem sicher und schnell zu öffnen sein, wobei eine synchrone Öffnung der einzelnen Kartuschen erstrebenswert ist.

Reaktive pastöse Zwei- oder Mehrkomponentensyteme müssen nach ihrer Herstellung bis zur Applikation getrennt aufbewahrt werden, um vorzeitige, unbeabsichtigte Reaktionen der Komponenten zu verhindern. Kartuschensysteme für die Applikation von pastösen Zwei- oder Mehrkomponentensystemen sind seit Jahrzehnten bekannt. Beispielhaft seien dafür die Dokumente CH 669 164 A5, EP 0 607 102 A1, EP 0 236 129 A2, DE 3 440 893 A1, US 4,690,306 A, US 2009/062808 A1, EP 0 787 535 A1, WO 2006/005 206 A1, EP 0 693 437 A1, EP 0 294 672 A, EP 0 261 466 A1 und EP 2 008 707 A1 genannt. Die Vermischung der pastösen Zwei- oder Mehrkomponentensysteme erfolgt unmittelbar bei der Applikation üblicherweise mit Hilfe von statischen Mischern. Exemplarisch seien dafür die Dokumente GB 1,188,516 A, US 2,125,245 A, US 5,968,018 A, US 4,068,830 A, US 2003/179648 A1, EP 0 664 153 A1 und EP 0 289 882 A1 angeführt. Nach der Befüllung der Kartuschen mit reaktiven Pasten müssen diese sicher bis zur Applikation verschlossen bleiben. Bewegliche Kolben, die auch zum Austragen des Kartuscheninhalts dienen, dichten dabei üblicherweise die Kartuschenböden ab. Zum Verschließen des Kartuschensystemkopfs des Kartuschensystems wurde eine Reihe von Lösungen vorgeschlagen. Ein einfaches, aber sehr wirksames Prinzip besteht darin, den Kartuschenkopf mit einem drehbaren Verschluss zu verschließen (EP 0 431 347 A1, DE 2 017 292 A1, US 3,215,298 A). Der Verschluss wird vor der Applikation herausgedreht. Anschließend wird ein Austragsrohr in ein Gewinde am Kartuschenkopf eingedreht oder mit einem, ein Gewinde nachbildendem Zapfensystem fixiert. Zwischen dem Öffnen der Kartuschen und dem Einsetzen des Austragsrohrs kann es, insbesondere wenn flüchtige Substanzen in den Pasten enthalten sind, zur Verdunstung von Bestandteilen der Pasten kommen.
Der gegenwärtig sehr häufig, insbesondere in der Klebstoff- und Dichtungsmittelindustrie verwendete Verschluss basiert darauf, dass am Kartuschenkopf das Wandmaterial der Kartusche sehr dünn ausgebildet ist, so dass diese Wandung leicht durchstochen werden kann.
Die Rückseite der Kartuschen wird üblicherweise durch bewegliche Kolben verschlossen, die zum Austreiben der Pasten bei der Applikation bestimmt sind. Bei feuchtigkeits- und luftempfindlichen Pasten können Aluminiumkartuschen verwendet werden, die mit Kunststoffkolben verschlossen sind und über die zur Abdichtung Aluminiumzylinder, die an einer Seite geschlossen sind, eingepresst werden. Bei der Applikation der Pasten wird der einseitig geschlossene Aluminiumzylinder zusammen mit dem Kolben mit Hilfe von Auspresspistolen nach vorne in Richtung Kartuschenkopf bewegt und die Paste dabei ausgetrieben. Bei medizinischen Anwendungen kann jedoch ein Kontakt von Pasten mit Aluminiumoberflächen problematisch sein. Bei der Verwendung von Kartuschensystemen für sterile pastöse Medizinprodukte ist es notwendig, dass nicht nur die Pasten sondern natürlich auch die Kartuschen und das sekundäre Packmittel in steriler Form dem medizinischen Anwender zur Verfügung gestellt werden. So können zum Beispiel nach aseptischer Befüllung der zuvor sterilisierten Kartuschen diese direkt in ein steriles Packmittel überführt werden. Weiterhin kann es bei bestimmten Produkten sinnvoll sein, die Oberflächen der befüllten Kartuschen zusammen mit den Packmitteln nach erfolgter Verpackung zu sterilisieren. Neben der Gammasterilisation, die bei polymerisierbaren Pastensystemen nicht angewendet werden kann, besteht die Möglichkeit mit dem Gas Ethylenoxid zu sterilisieren.

Problematisch bei Pastensystemen, die Monomere mit hohem Dampfdruck enthalten, ist bei einer solchen Sterilisation mit Gas jedoch, dass nach der eigentlichen Sterilisation, bei der Entfernung des restlichen Ethylenoxids durch Einwirkung von Vakuum, ein Teil der Monomere in den Kartuschen verdampft, die dabei eine Gasphase in den Kartuschen ausbilden und somit einen Druck gegen die Kolben ausüben können. Das bedeutet, dass die Kolben unerwünscht in Richtung Kartuschenböden bewegt werden und im Extremfall aus den Kartuschen ausgestoßen werden, so dass die Pasten ausfließen können.

Bei der Anwendung von Knochenzementen zur Fixierung von Totalgelenkendoprothesen muss immer berücksichtigt werden, dass bei diesen Operationen das OP-Personal unter zeitlichem Druck steht. Bei medizinischen Anwendungen von Kartuschensystemen für die Applikation von pastenförmige Polymethylmethacrylat-Knochenzemente sollten diese daher grundsätzlich so gestaltet sein, dass sie weitgehend resistent gegenüber Anwenderfehlern sind und auch in Stresssituationen schnell und sicher bedient werden können.

Ein essentieller Bestandteil von pastenförmigen Polymethylmethacrylat-Knochenzementen ist das Monomer Methylmethacrylat. Dieses Monomer verdampft leicht und hat bei Raumtemperatur einen recht hohen Dampfdruck. Bei der Verwendung von Methylmethacrylat enthaltenden Pasten muss deshalb unbedingt beachtet werden, dass bei Vakuumeinwirkung, wie bei der Entgasung im Rahmen der Sterilisation mit Ethylenoxid, die Kartuschenkolben in den Kartuschen durch das verdampfende Methylmethacrylat bewegt und im Extremfall aus den Kartuschen ausgestoßen werden können.

In der Medizin werden seit Jahrzehnten Polymethylmethacrylat-Knochenzemente zur dauerhaften mechanischen Fixierung von Totalgelenkendoprothesen eingesetzt. Diese basieren auf Pulver-Flüssigkeits-Systemen. Es wurden in jüngster Zeit auch Polymethylmethacrylat-Knochenzemente vorgeschlagen, die auf der Verwendung von Zementpasten beruhen (DE 10 2007 050 762 A1, DE 10 2008 030 312 A1, DE 10 2007 052 116A1). Für solche Ausgangskomponenten von Mischgütern sind Kartuschensysteme geeignet, die zumindest zwei Kartuschen mit jeweils einem Förderkolben umfassen. Um eine gleichmäßige Mischung der Ausgangskomponenten zu erreichen, ist ein paralleles Verschieben der Förderkolben notwendig. Hierfür wurde vorgeschlagen, die Förderkolben der beiden Kartuschen an den aus den Kartuschen herausragenden Enden zu verbinden.

EP 1 004 353 A1 beschreibt eine Vorrichtung zum Auspressen und dosierbaren Abgeben von fließfähigen Mehrkomponentenmassen unter Einwirkung eines Drucks auf flexible Beutel in zwei verschließbaren Aufnahmeräumen, die beide in einen in deinem Deckelement ausgebildten, die Mischeinrichtung aufnehmenden Mischraum münden.

Ein gattungsgemäßes Kartuschensystem mit zwei Kartuschen und zwei miteinander verbundenen Förderkolben ist aus der US 4,260,077 A bekannt. Das Kartuschensystem ist im Prinzip wie zwei direkt nebeneinander liegende Spritzen aufgebaut, deren Förderkolben am kartuschenbodenseitigen Ende mit einander verbunden werden können. An der Spitze des Kar tuschensystems ist eine gemeinsame Auslassöffnung für beide Kartuscheninhalte vorgesehen. Beim Auspressen der Inhalte werden die Förderkolben so weit in die Kartuschen gedrückt, bis die vorderen Enden der Förderkolben an die Kartuschenköpfe stoßen, beziehungsweise die Verbindung der Förderkolben auf die Kartuschenwände am Kartuschenboden trifft. Nachteilig ist hieran, dass die Förderkolben nicht durch eine Beaufschlagung des Kartuschenbodens mit Druck, also einem komprimiertem Gas, vorangetrieben werden können. Zum automatischen Antreiben der Förderkolben muss ein mechanischer Vortrieb bereitgestellt werden.

Aufgabe der Erfindung ist es also ein Kartuschensystem bereitzustellen, bei dem die Förderkolben auch mit Gasdruck bewegbar sind.

Diese Aufgabe wird dadurch gelöst, dass die zumindest zwei Förderkolben über zumindest einen Steg fest miteinander verbunden sind, die Kartuschenwände der durch den Steg oder die Stege verbundenen Kartuschen Schlitze umfassen, die parallel zur Kartuschenachse angeordnet sind, wobei die Breite der Schlitze zur Aufnahme des oder der Stege geeignet sind und bei einer Bewegung der Förderkolben in den Kartuschen sich der zumindest eine Steg durch Bereiche der Schlitze bewegt, wobei ein Mischraum parallel zwischen den Kartuschen angeordnet ist

Dabei kann vorgesehen sein, dass die Länge der Schlitze beginnend im Bereich des Kartuschenbodens bis mindestens zur Hälfte der Kartuschenlänge reicht.

Auch kann vorgesehen sein, dass die Kartuschenwände am Kartuschenboden durch die Schlitze vollständig geschlitzt sind.

Der Mischraum, der parallel zwischen den Kartuschen angeordnet ist, ist insbesondere derart angeordnet, dass die Kartuschenwände bereichsweise mit Mischraumwänden des Mischraums in einem ausgeführt sind, wobei im Bereich der Schlitze auch die Mischraumwände geschlitzt sind.

Dabei kann vorgesehen sein, dass im Inneren des Mischraums eine bewegliche Stange parallel zu den Förderkolben angeordnet ist, die mit den Förderkolben über zumindest einen Steg fest verbunden ist, wobei die Stange an der der Auslassöffnung zugewandten Seite ein Rastmittel umfasst und im Mischraum ein Gegenrastmittel angebracht ist, das mit dem Rastmittel der Stange derart zusammenwirkt, dass eine Bewegung der Stange aus dem Mischraum heraus und damit der Förderkolben aus den Kartuschen heraus deutlich erschwert, insbesondere verhindert ist.

Auch kann vorgesehen sein, dass die Kartuschen jeweils zumindest eine Öffnung in den Kartuschenwänden umfassen, die die Kartuschen mit dem Mischraum verbinden, dass im Mischraum oder am Kartuschenkopf ein im Mischraum verschiebbar angeordneter Verschluss oder ein am Kartuschenkopf drehbar gelagerter Verschluss derart angeordnet ist, dass er die Öffnungen der Kartuschen in einer Ausgangsposition verschließt und dass die Öffnungen in einer Endposition des Verschlusses zumindest bereichsweise geöffnet sind, wobei der Verschluss von der Ausgangsposition zur Endposition verschiebbar oder drehbar ist.

Dabei kann es vorteilhaft sein, dass im Mischraum ein Anschlag, vorzugsweise in Form von Zapfen oder Stegen, angeordnet ist, der die Bewegung des verschiebbaren Verschlusses im Mischraum begrenzt und dadurch die Endposition des verschiebbaren Verschlusses definiert. Mit der Erfindung wird auch vorgeschlagen, dass die Auslassöffnung in einem Kartuschensystemkopf angeordnet ist, und der Kartuschensystemkopf ein Befestigungsmittel zum Befestigen eines Austragsrohrs umfasst, insbesondere auf der Innenseite des Kartuschensystemkopfs, vorzugsweise ein Gewinde oder mehrere Zapfen.

Nach einer weiteren Ausgestaltung des erfindungsgemäßen Kartuschensystems kann vorgesehen sein, dass die Förderkolben die Kartuschen dicht, insbesondere gasdicht, verschließen. Erfindungsgemäße Kartuschensysteme können sich des Weiteren dadurch auszeichnen, dass der Steg oder zumindest einer der Stege bodenseitig mit den Förderkolben fest verbunden ist, und ein Arretierungsmittel mit dem zumindest einen Steg verbunden ist, wobei das Arretierungsmittel einen Bereich parallel oder zwischen den Kartuschen reicht und dort in ein Gegenarretierungsmittel greift, so dass eine Bewegung des zumindest einen Stegs und der Förderkolben in Richtung des Kartuschenbodens blockiert ist.

Dabei kann vorgesehen sein, dass in dem Bereich eine Entarretiervorrichtung vorgesehen ist, die fest mit dem verschiebbaren Verschluss verbunden ist, so dass beim Verschieben des verschiebbaren Verschlusses in seine Endpostition, eine Entarretierung der Arretierungsmittel erfolgt, so dass eine Bewegung des zumindest einen Stegs und der Förderkolben in Richtung des Kartuschenbodens möglich ist.

Es wird des Weiteren vorgeschlagen, dass die Förderkolben die Schlitze auf der Innenseite der Kartuschen abdecken.

Schließlich kann vorgesehen sein, dass die Förderkolben und die Kartuschenwände gasdicht und miteinander schlüssig aufgebaut sind, so dass durch einen Gasdruck auf den Kartuschenboden die Förderkolben in die Kartuschen drückbar sind.

Dem erfindungsgemäßen Kartuschensystem liegt die überraschende Erkenntnis zugrunde, dass durch Schlitze in den Kartuschenwänden ein Steg zur Verbindung der Förderkolben verwendet werden kann, der bei einer Bewegung der Förderkolben in die Kartuschen hinein, durch die in den Kartuschenwänden vorgesehenen Schlitze hindurch bewegt wird. Dadurch kann der Steg flach im Bereich des Kartuschenbodens an den Förderkolben angeordnet werden. Der Steg muss nicht direkt am Kartuschenboden beziehungsweise an den dem Kartuschenboden zugewandten Enden der Förderkolben an den Förderkolben befestigt sein, sondern kann auch ein Stück weiter in Richtung Zentrum des Kartuschensystems verlegt werden. Dadurch, dass die Förderkolben in die Kartuschen hinein gedrückt, beziehungsweise versenkt werden, also bei dieser Anordnung nicht aus den Kartuschen heraus ragen, ist eine Bewegung der Förderkolben mit Druckluft möglich.

Ein solches Kartuschensystem kann komplett aus kostengünstigen Kunststoffspritzgussteilen gefertigt werden. Das Kartuschensystem ermöglicht es, bei Krafteinwirkung in Richtung Kartuschensystemkopf die Kolben synchron in den Kartuschen zu bewegen und damit die Pasten gleichmäßig auszupressen, damit das Mischungsverhältnis der Pasten zueinander gewährleistet wird.

Eine weitere Vereinfachung und Sicherstellung der Bedienbarkeit eines erfindungsgemäßen Kartuschensystems ergibt sich dadurch, dass eine Bewegung der Förderkolben bei Einwirkung von Vakuum durch eine Arretierung sicher verhindert werden kann.

Unter dem Mischraum ist im Sinne der vorliegenden Erfindung, der Bereich zu verstehen, der sich zwischen den wenigstens zwei Kartuschen befindet, sowie die daran angrenzenden Bereiche. Er erstreckt sich vom Kartuschenboden, d. h. hinteren, bodenseitigen Teil des Kartuschensystems, bis zur Auslassöffnung am Kartuschensystemkopf an der Vorderseite des Kartuschensystems, umfasst also auch den Innenraum des Kartuschensystemkopfs. Nicht überall im Mischraum muss dabei die Mischung der Ausgangskomponenten des Mischguts erfolgen, sondern nur in Teilbereichen desselben. Ein Mischraum der mit Schläuchen an die Kartuschen angeschlossen ist, und daher der Mischraum nicht im geometrischen Sinne genau zwischen den Kartuschen liegt, ist ebenfalls erfindungsgemäß.

Unter einem verschiebbaren Verschluss im Sinne der vorliegenden Erfindung ist auch ein Verschluss zu verstehen, der zunächst in der Ausgangsposition durch eine feste Verbindung mit den Wänden des Zwischenraums, beispielsweise durch dünne Brücken, verbunden ist, sofern diese feste Verbindung Sollbruchstellen aufweist, die bei einer Krafteinwirkung auf den Verschluss brechen und so das Verschieben des Verschlusses unter Krafteinwirkung ermöglichen. Dabei wird vorgeschlagen, dass die feste Verbindung oder die festen Verbindungen mit den Sollbruchstellen von den Öffnungen der Kartuschen aus gesehen in der Richtung vorgesehen sind, in die sich der Verschluss, beim Öffnen bewegt, um einen Eintrag von Partikeln der Bruchstellen in das Mischgut bzw. in die Ausgangskomponenten des Mischguts zu verhindern. Als Endposition des Verschlusses im Sinne der Erfindung ist keine endgültige Endposition zu verstehen. So wäre es beispielsweise vorstellbar vorzusehen, dass der verschiebbare Verschluss auch von der Endposition in die Ausgangsposition bewegbar ist und die Kartuschen des Kartuschensystems dann erneut befüllbar sind. Dadurch ist das Kartuschensystem wiederverwendbar. Ebenso ist dann die Ausgangsposition, wie die Endposition, auch nur als eine von zumindest zwei Positionen zu verstehen, in die der verschiebbare Verschluss verschiebbar ist. Im Folgenden werden Ausführungsbeispiele der Erfindung anhand von sechzehn schematisch dargestellten Zeichnungen erläutert. Dabei zeigt:
- Figur 1:: eine Querschnittansicht in Längsrichtung eines erfindungsgemäßen Kartuschensystems;
- Figur 2:: eine Aufsicht auf die Vorderseite eines erfindungsgemäßen Kartuschensystems mit der Auslassöffnung nach Figur 1;
- Figur 3:: eine Aufsicht auf den Kartuschenboden eines erfindungsgemäßen Kartuschensystems nach Figur 1;
- Figur 4:: eine Querschnittansicht eines Kartuschensystems nach Figur 1 entlang des Schnitts A-A in Figur 1;
- Figur 5:: eine Aufsicht auf einen Kartuschenboden eines zweiten erfindungsgemäßen Kartuschensystems;
- Figur 6:: eine Querschnittansicht in Längsrichtung eines Austragsrohrs für ein solches Kartuschensystem; und
- Figur 7:: ein drittes erfindungsgemäßes Kartuschensystem mit einer am Verschluss angebrachten Entarretier-Vorrichtung in Querschnittansicht.

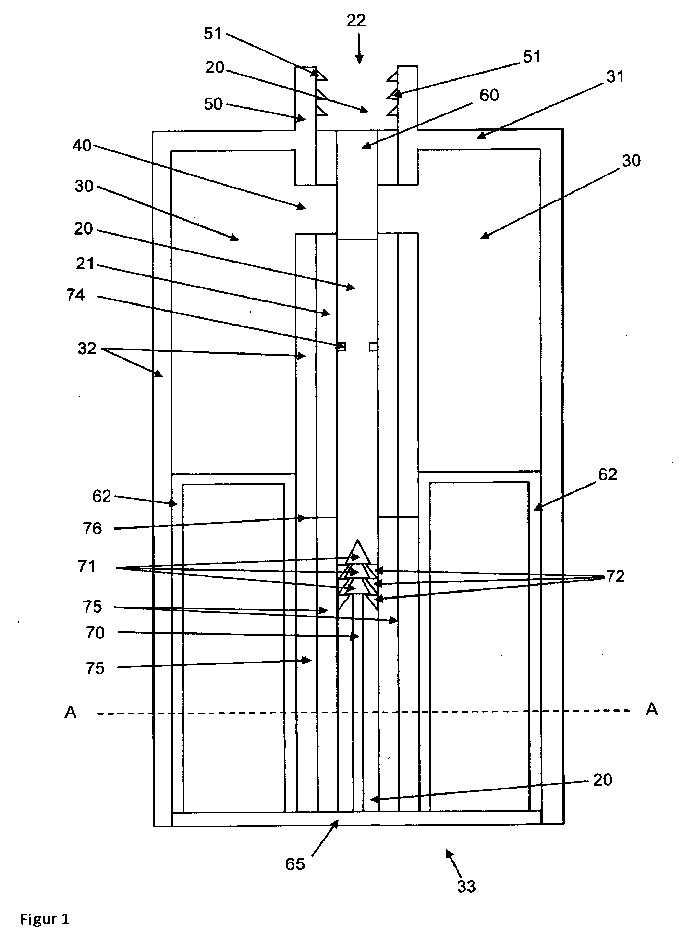

Figur 1 zeigt eine Querschnittansicht eines erfindungsgemäßen Kartuschensystems (1) mit einem Mischraum (20), der durch Mischraumwände (21) begrenzt ist und in eine Auslassöffnung (22) mündet. Der Mischraum (20) erstreckt sich im Bereich zwischen zwei Kartuschen (30) und ist als zylindrischer Hohlkörper ausgebildet. Die Kartuschen (30) sind durch Kartuschenköpfe (31), Kartuschenwände (32) und einen Kartuschenboden (33) begrenzt. Im Inneren der Kartuschen (30) befinden sich die Ausgangskomponenten (nicht gezeigt) für ein zu mischendes Mischgut. In den Kartuschenwänden (32) und den Mischraumwänden (21) befinden sich Öffnungen (40), die das Innere der Kartuschen (30) mit dem Inneren des Mischraums (20) verbinden. Dieses Kartuschensystem (1) ist also zum Mischen eines Mischguts bestehend aus zwei Komponenten geeignet.

Die Auslassöffnung (22) ist in einem Kartuschensystemkopf (50) gebildet, der auf der Innenseite ein Befestigungsmittel (51) in Form eines Gewindes oder in Form von Zapfen, die ebenfalls ein Gewinde bilden können, umfasst.

Ein verschiebbarer Verschluss (60) in Form eines massiven Zylinders steckt in Presspassung im oberen, dass heißt in dem der Auslassöffnung (22) zugewandten Ende des Mischraums (20). Der verschiebbare Verschluss (60) verschließt die Öffnungen (40) die das Innere der Kartuschen (30) mit dem Inneren des Mischraums (20) verbinden. Der verschiebbare Verschluss (60) kann aber ebenso eine geschlossene Mantelfläche aufweisen.

Die Kartuschen (30) sind in Richtung der Kartuschenböden (33) mit Förderkolben (62) verschlossen. Die in Längsrichtung der Kartuschen (30) verschiebbaren Förderkolben (62) können mit technisch üblichen Abstreiflippen und Dichtungen an den dem Kartuschenkopf (31) zugewandten Enden der Förderkolben (62) ausgerüstet sein, um einen dichten Verschluss der Kartuschen (30) zu gewährleisten.

Am Kartuschenboden (33) sind die Förderkolben (62) durch einen Steg (65) oder eine Platte (nicht gezeigt) miteinander verbunden. Ein zusätzlicher Steg, der durch die Mischraumwände (21) und durch die Kartuschenwände (32) reicht, kann ein Stück weiter in Richtung des Inneren des Kartuschensystems (1) vorgesehen sein, um der Anordnung mehr Stabilität und Führungssicherheit zu geben.

Mit den Stegen (65) ist zusätzlich eine Stange (70) fest verbunden, die in das den Kartuschenböden (33) zugewandten Ende des Mischraums (20) ragt. An der Spitze der Stange (70) sind Rastmittel (71) angebracht. An den Innenwänden (21) des Mischraums (20) sind Gegenrastmittel (72) angebracht, die in das Rastmittel (71) greifen. Die Gegenrastmittel (72) sind aus einem flexiblen Material, wie zum Beispiel Gummi, Gefertigt. Die Rastmittel (71) können aus einem normalen Kunststoff gefertigt sein. Wenn die Stange (70) so tief in den Mischraum (20) eingeschoben ist, dass die Rastmittel (71) in die Gegenrastmittel (72) greifen, wird eine Bewegung der Stange (70) aus dem Mischraums (20) heraus verhindert. Gleichzeitig ist es aber ohne weiteres möglich, die Stange (70) tiefer in den Mischraum (20) zu schieben.

Im Mischraum (20) befindet sich also die Stange (70), die in Längsrichtung der Förderkolben (62) so angeordnet ist, dass sie an den Stegen (65) mit einem Ende befestigt ist, und eine Länge von mindestens der Länge der Förderkolben (62) hat. Wesentlich ist, dass die Stange (70) an der dem Kartuschenkopf (31) zugewandten Seite gezahnt ist. Die Zähne sind so orientiert, dass die Spitzen der Zähne in Richtung Kartuschenboden (33) zeigen. Die Stange (70) hat einen Querschnitt kleiner dem Querschnitt des Mischraums (20).

Am Ende des Mischraums (20) ist an der dem Kartuschenboden (33) zugewandten Seite, eine flexible Rastung (72) angeordnet, die einen Querschnitt kleiner oder gleich der gezahnten Stange (70) hat. Das bedeutet, die gezahnte Stange kann in diese Rastung (72) einrasten. Die Rastung (72) ist so angeordnet, dass nur eine Bewegung der gezahnten Stange (70) in Richtung Kartuschenkopf (31) möglich ist. Eine Rückwärtsbewegung in Richtung Kartuschenboden (33) ist nicht möglich.

Durch die gezahnte Stange (70) und die Rastung (72) wird die Position der Förderkolben (62) in den Kartuschen (30) fixiert, so dass eine Rückwärtsbewegung der Förderkolben (62) aus den Kartuschen (30) auch bei der Einwirkung von Vakuum sicher verhindert wird.

Der verschiebbare Verschluss (60) kann durch die Einwirkung einer Kraft durch die Auslassöffnung (22) in Richtung des Kartuschenbodens (33) verschoben werden. Die Kraft kann durch einsetzen eines geeigneten Austragsrohrs (nicht gezeigt) in die Auslassöffnung (22) erfolgen. Ein Anschlag (74), der in Form von Zapfen an den Innenwänden (21) des Mischraums (20) angebracht ist, verhindert, dass der verschiebbare Verschluss (60) tiefer als bis zum Anschlag (74) in den Mischraum (20) eingeschoben werden kann. Dadurch definiert der Anschlag (74) die Endposition des verschiebbaren Verschlusses (60).

Um eine Bewegung der Stege (65) durch die Mischraumwände (21) und die dem Mischraum (20) zugewandten Kartuschenwände (32) hindurch zu ermöglichen, sind diese mit einem Schlitz (75) versehen, der von den Kartuschenböden bis zum Schlitzende (76) im Inneren des Kartuschensystems (1) reicht. Durch diesen Schlitz (75) ist eine Bewegung der Stege (65) ohne weiteres möglich. Dadurch kann bei einer Beaufschlagung des Kartuschbodens (33) mit Gasdruck erreicht werden, dass die Förderkolben (62) ins Innere der Kartuschen (30) gedrückt werden. Durch die Begrenzung der Kartuschenwände (32) wird der Gasdruck auf den Böden der Förderkolben (62) auch dann noch aufrechterhalten, wenn sich diese bereits tief im Inneren der Kartuschen (30) befinden.

Die Bewegung des Systems Förderkolben (62), Stange (70) mit Rastmittel (71) und Steg (65) wird erst dann zum Halten kommen, wenn der Steg (64) das Schlitzende (76) erreicht. Es ist wichtig darauf hinzuweisen, dass das erfindungsgemäße Kartuschensystem (1) ohne weiteres auch ohne den Verschluss (60), den Mischraum (20) mit den Mischraumwänden (21), die Stange (70) mit Rastmittel (71) und den Kartuschenkopf (50) realisierbar ist. Für Figur 1 bedeutet dies, dass der mittlere Bereich auch weggelassen werden kann, sofern die Auslassöffnung (22) erhalten bleibt. Es reicht also völlig aus, zwei parallele Kartuschen (30) direkt nebeneinander, miteinander in Verbindung mit einer gemeinsamen Auslassöffnung (22) vorzusehen, die im Bereich des Kartuschenbodens (33) fest mit einem Steg (65) miteinander verbunden sind, wobei für die Bewegung des Stegs (65) Schlitze (75) in den Kartuschenwänden (32) vorgesehen sind.

Ein Gasdruck aus Richtung des Kartuschenbodens (33) muss auch auf die bodenseitigen Bereiche der Schlitze (75) drücken und die Schlitze (75) dürfen nicht derart mit der Umgebung in Verbindung stehen, dass ein auf die Förderkolben (62) lastender Druck, wenn diese bereits teilweise in die Kartuschen (30) eingeschobenen sind, entweichen kann. Am besten sind die Schlitze (75) vollständig, gas- und druckdicht von der Umgebung getrennt. Ebenso sollten die Förderkolben (62) gegenüber dem Inneren der Kartuschen (30) gas- und druckdicht getrennt sein. Solche Druckdichtungen (nicht gezeigt) für bewegliche Teile sind handelsüblich und leicht erhältlich.

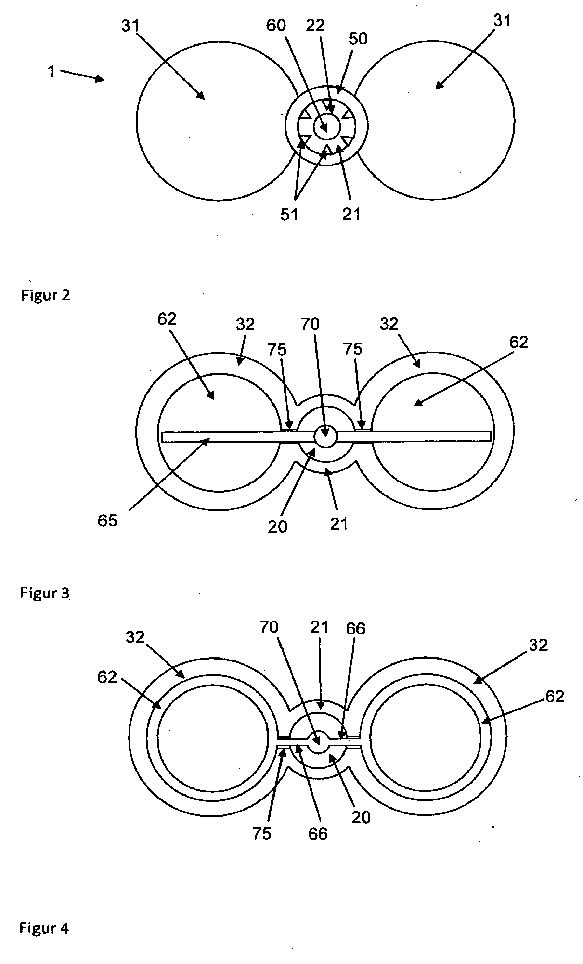

Figur 2 zeigt eine Aufsicht auf ein solches Kartuschensystem (1) nach Figur 1 und zwar auf den Kartuschensystemkopf (50). Ein Blick in die Auslassöffnung (22) zeigt die Mischraumwände (21), die Befestigungsmittel (51) und in der Mitte den verschiebbaren Verschluss (60). Auf beiden Seiten des Kartuschensystemkopfs (50) sind die Kartuschenköpfe (31) der darunter liegenden Kartuschen (30) (in Figur 2 nicht zu erkennen) angebracht.

Figur 3 zeigt eine Aufsicht auf ein solches Kartuschensystem (1) aus Richtung des Kartuschenbodens (33). Der Mischraum (20), der durch die Mischraumwände (21) begrenzt ist, wird auf beiden Seiten von den Kartuschen (30), von denen nur die Kartuschenwände (32) und die Förderkolben (62) zu sehen sind, flankiert. In der Mitte des in dieser Richtung offenen Mischraums (20) befindet sich die Stange (70), die ins Innere des Mischraums (20) ragt. Die Stange (70) und die Förderkolben (62) sind durch den Steg (65) miteinander fest verbunden. Um eine Bewegung des Stegs (65) ins Innere des Kartuschensystems (1) bei einer Bewegung der Förderkolben (62) in die Kartuschen (30) hinein zu ermöglichen, ist in den Wänden (21, 32) des Mischraums (20) und der Kartuschen (30) ein Schlitz (75) vorgesehen.

Figur 4 zeigt eine Querschnittansicht eines solchen Kartuschensystems (1) entlang des Schnitts (A-A) in Figur 1. Zwischen den beiden Kartuschen (30), die durch die Förderkolben (62) und die Kartuschenwände (32) erkennen sind, befindet sich der Mischraum (20), der durch die Mischraumwand (21) begrenzt ist. Die Förderkolben (62) sind als beidseitig geschlossene Hohlzylinder aufgebaut. Im Inneren des Mischraums (20) ist die Stange (70) angeordnet. Die Stange (70) und die Förderkolben (62) sind durch den Steg (65) miteinander verbunden. In den die Kartuschen (30) mit dem Mischraum (20) verbindenden Wänden (21, 32) sind Schlitze (75) vorgesehen, die eine Bewegung des Stegs (65), der die Förderkolben (62) mittig verbindet, im Inneren des Kartuschensystems (1) ermöglichen. Ein zusätzlicher Steg (nicht zu sehen) kann weiter im Inneren des Kartuschensystems (1) vorgesehen sein, der sich ebenfalls durch die Schlitze (75) bewegen lässt.

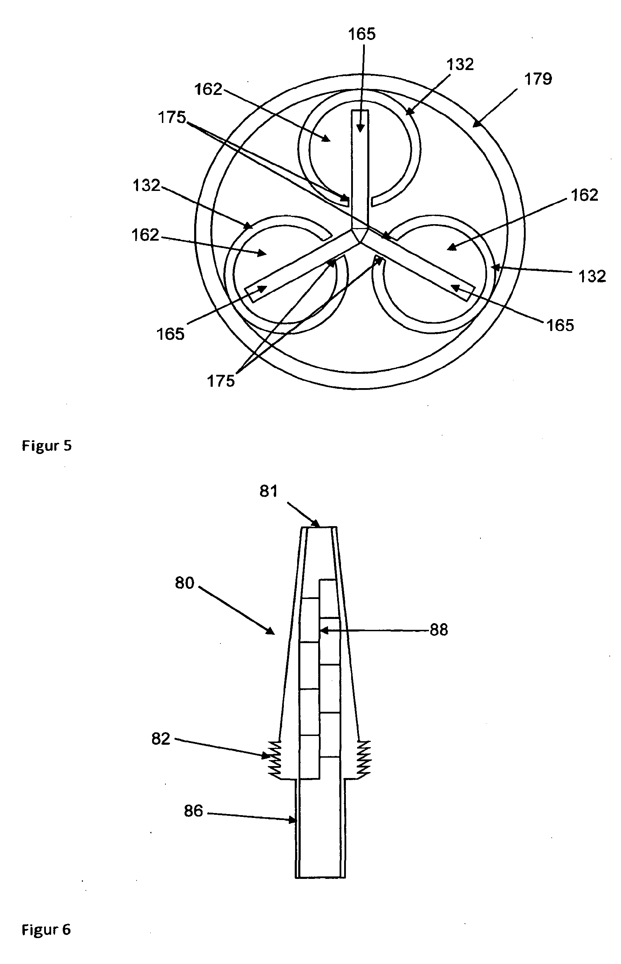

Figur 5 zeigt eine Aufsicht auf einen Kartuschenboden eines alternativen Kartuschensystems zum Mischen eines Mischguts bestehend aus drei Komponenten. Zu diesem Zweck umfasst das Kartuschensystem drei Kartuschen, von denen aus Richtung des Kartuschenbodens nur die Kartuschenwände (132) und die Förderkolben (162) zu erkennen sind. Zwischen den Kartuschenwänden (132) befindet sich ein Freiraum. Dort könnte auch ein geschlossener Mischraum angeordnet sein. Die Förderkolben (162) sind mit Stegen (165) miteinander fest verbunden. Das gesamte Kartuschensystem ist durch eine zusätzliche Ummantelung (179) eingeschlossen, der die Kartuschen über eine Verbindung zu den Kartuschenwänden (132) positioniert.

In den Kartuschenwänden (132) sind Schlitze (175) vorgesehen, die breiter als die Dicke der Stege (165) sind. Es reicht aus, wenn die Breiten der Schlitze (175) gleich den Dicken der Stege (165) sind. Es kommt dann zwar zu einer Reibung zwischen den Stegen (165) und den Schlitzwänden, die jedoch unkritisch ist, sofern die auf die Förderkolben (162) ausgeübte Kraft größer als die Reibungskräfte ist. Die Ummantelung (179) kann am Kartuschenkopf (nicht zu erkennen) geschlossen sein, so dass ein Gasdruck auf den gesamten Querschnitt der Ummantelung (179) ausgeübt werden kann. Dadurch übt der Gasdruck eine Kraft auf die Förderkolben (162) aus. An der im Bereich der Kartuschenköpfe angeordneten Auslassöffnung (nicht gezeigt), mit der alle Kartuschen verbunden sind, wirkt nur der Umgebungsdruck. Durch die Druckdifferenz zwischen der Rückseite aus Richtung des Betrachters und der Vorderseite Richtung Auslassöffnung werden die Förderkolben (162) bewegt.

Figur 6 zeigt ein Austragsrohr (80) für ein erfindungsgemäßes Kartuschensystem (1) in Querschnittansicht. Am oberen Ende des Austragsrohrs (80) befindet sich die Austragsrohrspitze (81), die zum Applizieren eines gemischten Mischguts dient. Darunter befindet sich ein Außengewinde (82), mit dem das Austragsrohr (80) in das Innengewinde (51) des Kartuschensystems (1) eingeschraubt werden kann. Öffnungen (nicht gezeigt) können in einer darunter angeordneten Verlängerung (86) vorgesehen sein.

Die Verlängerung (86) hat gleichen oder einen geringeren Außendurchmesser als der Durchmesser des Mischraums (20). Dadurch kann, wenn das Austragsrohr (80) in den Kartuschensystemkopf (50) hinein gedreht wird, die Verlängerung (86) jenseits des Kartuschensystemkopfs (50) in den Mischraum (20) eindringen und so den verschiebbaren Verschluss (60) tiefer in den Mischraum (20) hinein schieben. Dadurch werden die Öffnungen (40) zum Mischraum (20) geöffnet. Die Verlängerung (86) ist als Hohlzylinder ausgebildet, und die Öffnungen dienen dazu, die zu mischenden Ausgangskomponenten, die durch die Öffnungen (40) in den Kartuschenwänden (32) hindurch passieren, nicht zu blockieren. Die zu mischenden Materialien passieren also sowohl die Öffnungen (40), als auch die Öffnungen. Im Inneren des Austragsrohrs (80) ist ein statischer Mischer (88) vorgesehen.

Eine Verlängerung (86) könnte aber auch durch eine Stange, die in einem T-Stück endet, gebildet werden, die beispielsweise als eine Verlängerung der zentralen Achse des statischen Mischers (88) ausgebildet ist. Eine solche Verlängerung (86) würde dann keine zusätzlichen Öffnungen benötigen. Auch könnte eine Verlängerung des statischen Mischers (88) über das Gewinde (82) hinaus als Verlängerung (86) dienen.

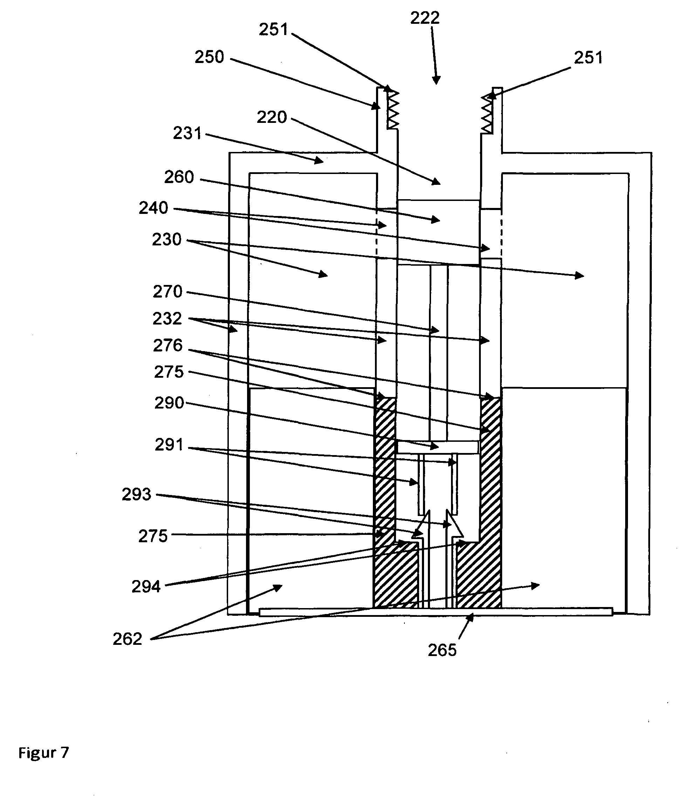

Figur 7 zeigt ein drittes Ausführungsbeispiel eines erfindungsgemäßen Kartuschensystems in schematischer Querschnittansicht. Bei diesem Ausführungsbeispiel ist an einem Verschluss (260) eine Stange (270) angeordnet, die von einer Zentriervorrichtung (290) im Zentrum eines als Hohlkörper ausgeformten Mischraums (220) ausgerichtet ist. An der Stange (270) beziehungsweise an deren Zentriervorrichtung (290) ist eine Entarretiervorrichtung (291) in Form eines Hohlzylinders angeordnet. Der Verschluss (260) verschließt Öffnungen (240) in den Kartuschenwänden (232), die die Kartuschen (230) mit den Mischraum (220) verbinden. Am vorderen Ende des Kartuschensystems befindet sich am Kartuschenkopf (231) ein Kartuschensystemkopf (250) mit einem Befestigungsmittel (251) zum Befestigen eines Austragsrohrs. Der Kartuschensystemkopf (250) ist als Hohlzylinder aufgebaut, der im Inneren eine Auslassöffnung (222) bildet.

Bodenseitig sind die Kartuschen (230) durch Förderkolben (262) verschlossen. Die Förderkolben (262) sind über einen Steg (265) miteinander verbunden. In den Kartuschenwänden (232) sind Schlitze (275) vorgesehen, deren Breite ausreicht, um einen Bewegung des Stegs (265) durch die Schlitze (275) zu ermöglichen. Der Bereich, in dem die Kartuschenwände (232) geschlitzt sind, reicht bis zum Schlitzende (276) im Inneren des Kartuschenssystems und ist schraffiert dargestellt.

Zwei Arretierungshaken (293) sind mit dem Steg (265) verbunden. Anstatt der Arretierungshaken (293) können auch andere Arretierungsmittel (293) eingesetzt werden. Die Arretierungshaken (293) sind aus einem flexiblen Material aufgebaut, so dass sie relativ leicht zusammen gedrückt werden können. Im Bereich der Arretierungshaken (293) ist im Mischraum (220) eine Arretierungsstufe (294) angeordnet, so dass die Haken der Arretierungshaken (293) in die Arretierungsstufen (294) greifen können.

Im Ausgangszustand des Verschlusses (260) verschließt dieser die Öffnungen (240). Die Arretierungshaken (293) verhindern eine Bewegung des Stegs (265) und damit der an dem Steg (265) befestigten Förderkolben (262) aus den Kartuschen (230) heraus.

Wird ein Austragsrohr mit Verlängerung, wie das nach Figur 6, in die Auslassöffnung (222) eingebaut, so werden die Öffnungen (240) freigelegt und die Entarretiervorrichtung (291) wird so über die Arretierungshaken (293) geschoben, dass diese zusammengedrückt werden und so eine Bewegung des Stegs (265) von der Auslassöffnung (222) weg und damit der Förderkolben (262) aus den Kartuschen (230) heraus möglich wird.

Gleichzeitig mit dem Einsetzen des Austragsrohrs wird also der an der Zentriervorrichtung (290) angeordnete Hohlzylinder (291) über die Arretierungshaken (293) geschoben, wobei sich diese senkrecht zur Längsachse des Mischraums (220) biegen, und wobei sich die Sperrzapfen Arretierungshaken (293) von der Arretierungstufe (294) weg bewegen und damit die Verriegelung des Stegs (265) entriegelt wird.

Eine Variante der Erfindung ist, dass an der dem Kartuschensystemkopf (250) zugewandten Seite des Mischraums (220) ein Innengewinde (251) oder ein Schnappverschluss angeordnet ist, dass sich im Mischraum (220) ein in Längsrichtung der Kartuschen (230) verschiebbarer Verschluss (260) in Presspassung befindet, der an der dem Kartuschensystemkopf (250) zugewandten Seite über seiner Fläche senkrecht zur Längsachse des Hohlraums (220) geschlossen ist, dass der verschiebbare Verschluss (660) über den Öffnungen (240) der Kartuschen (230) angeordnet ist, das der verschiebbare Verschluss (260) über eine Stange (270) mit einer kreisförmigen oder sternförmigen Zentrierscheibe (290) verbunden ist, an deren Unterseite ein Hohlzylinder (291) angeordnet ist, dass der Hohlzylinder (291) einen kleineren Durchmesser als der Innendurchmesser des Mischraums (220) besitzt, dass an einem Ende eines einsetzbaren Austragsrohrs ein Außengewinde angeordnet ist oder ein Hohlzylinder mit Zapfen für eine Schnappung und dass am Ende des Außengewindes oder des Zylinders mit Zapfen in Längsrichtung eine am Zylindermantel perforierte, also mit Öffnungen versehene Verlängerung in Form eines Hohlzylinders oder eines anderen in Längsrichtung offenen, in der Mantelfläche perforierter Hohlkörpers angeordnet ist.

Der Vorteil dieser Anordnung besteht darin, dass zeitgleich mit dem Einsetzen des Austragsrohrs die Öffnung der Kartuschen (230) erfolgt und gleichzeitig auch die durch den Steg (265) mit den Förderkolben (262) verschlossenen Kartuschenböden frei gegeben werden. Dadurch kann der Anwender erst dann die mit den Ausgangskomponenten gefüllte Kartuscheneinsätze in die entsprechende Auspresspistole/Zementpistole, mit der das Kartuschensystem betrieben wird, einsetzen, wenn sowohl das Austragsrohr eingesetzt ist, als auch der zum Verschluss der Kartuschenböden eingesetzte Steg (265) mit den Förderkolben (262) entriegelt und entfernt ist. Es ist somit unmöglich, Kartuscheneinsätze in Auspresspistole oder Zementpistolen zum Bedienen des Kartuschensystems einzusetzen, ohne dass das Austragsrohr eingesetzt ist und die Kartuschen (230) geöffnet sind. Dadurch ist eine Fehlbedienung nahezu unmöglich.

In gleicher Weise, wie sich der Steg (265) durch den Schlitz (275) bewegen lässt, bewegt er sich auch durch die Arretierungshaken (293), die Entarretiervorrichtung (291), die Zentriervorrichtung (290) und die Stange (270) hindurch, und/oder an denen vorbei. Ebenso könnten sich die Arretierungshaken (293) mit dem Steg (265) zusammen durch diese Teile hindurch und/oder an diesen Teilen vorbei bewegen, wenn die Arretierungshaken (293) durch die Entarretiervorrichtung (291) verformt wurden. Es kann auch vorgesehen sein, dass die Arretierungshaken (293) bei der Entarretierung abgebrochen werden.

Alle beschriebenen Ausführungsformen lassen sich problemlos auf Kartuschensysteme mit drei, vier oder noch mehr Kartuschen, zur Mischung eines Mischguts aus drei, vier oder noch mehr Komponenten übertragen. Bei der Verwendung von sehr vielen Komponenten, sind die Kartuschen zweckmäßigerweise nicht mit zylindrischer Form auszugestalten, sondern in Form von Segmenten.

Unter dem Begriff Kartuschensystem werden Kartuschen verstanden, die aus zwei, drei, vier, fünf oder mehreren Kartuschen (30, 230) aufgebaut sind, wobei die einzelnen Kartuschen (30, 230, 330) parallel zueinander angeordnet sind. Die Kartuschen (30, 230) können zylinderförmig aufgebaut sein oder Hohlkörper mit einer anderen axialen Symmetrie sein. Es handelt sich um sogenannte Side-by-side-Kartuschen. Die Kartuschen können bereits mit Ausgangskomponenten für ein zu mischendes Mischgut gefüllt sein, können aber auch leer sein, d. h. dass sie noch mit Inhalt, z. B. Kartuscheneinsätzen gefüllt werden müssen.

Der verschiebbare Verschluss (60, 260) hat bevorzugt eine geschlossene Mantelfläche. Wesentlich ist, dass die Kartuschen (30, 230) mit Förderkolben (62, 162, 262) verschlossen sind, wobei die Förderkolben (62, 162, 262) an der dem Kartuschenboden (33) abgewandten Seite durch Stege (65, 165, 265) miteinander verbunden sind. Das bedeutet, dass die Förderkolben (62, 162, 262) eine Einheit bilden und nicht einzeln bewegt werden können. Dadurch ist ein gleichmäßiges Austreiben der Pasten aus allen Kartuschen (30, 230) des Kartuschensystems möglich. Diese Eigenschaft ist essentiell, wenn die Kolben durch direkte Beaufschlagung mit komprimiertem Gas bewegt werden sollen.

Der Vorteil einer erfindungsgemäßen Ausgestaltung des Kartuschensystems besteht auch darin, dass alle Öffnungen (40, 240) in den Kartuschen (30, 230) nur durch einen verschiebbaren Verschluss (60, 260) verschlossen sind. Dieser hat einen geringfügig größeren Querschnitt als der Mischraum (20, 220). Das bedeutet, dass die Mantelfläche des verschiebbaren Verschlusses (60, 260) an die Innenwand des Mischraums (20, 220) bzw. dessen Wände (21) gepresst wird. Wenn der verschiebbare Verschluss (60, 260) über den Öffnungen (40, 240) der Kartuschen (30, 230) liegt, so sind diese verschlossen. Dabei überlappt der verschiebbare Verschluss (60, 260) die Öffnungen (40, 240) vollständig in der Weise, dass noch eine für die Dichtung genügende, geschlossene Mantelfläche an der Innenwand (21) des Mischraums (20, 220) anliegt. Die Dichtung wird infolge des Anpressdrucks durch den engen Kontakt der Mantelfläche des verschiebbaren Verschlusses (60, 260) mit der Innenwand (21) des Mischraums (20, 220) erreicht.

Viele der in der Medizin üblichen Kunststoffe, wie Polypropylen, haben nur geringe Haftreibungs- und Gleitreibungskoeffizienten. Das bedeutet, dass bei Gleitpaarungen solcher Kunststoffe durch relativ geringe Krafteinwirkung Gleitvorgänge bewirkt werden können. Der verschiebbare Verschluss (60, 260) kann daher bei Verwendung von geeigneten Kunststoffen durch axiale Bewegung verschoben werden. Dadurch ist es möglich, den verschiebbaren Körper von den Öffnungen (40, 240) der Kartuschen (30, 230) weg zu bewegen und damit die Kartuschen (30, 230) zu öffnen. Wenn sich der verschiebbare Verschluss (60, 260) axial bewegt, können somit synchron die Öffnungen (40, 240) aller Kartuschen (30, 230) gleichzeitig geöffnet werden.

Beim Eindrehen des Austragsrohrs (80) bewegt sich die Verlängerung (86) mit in Richtung Kartuschenboden (33). Dadurch wird auf den verschiebbaren Verschluss (60, 260) eine Kraft in Richtung Kartuschenboden (33) ausgeübt. Sobald die Haftreibung zwischen der Mantelfläche des verschiebbaren Verschlusses (60, 260) und der Innenwand (21) des Mischraums (20, 220) überwunden ist, kann sich der verschiebbare Verschluss (60, 260) in Richtung Kartuschenboden (33) bewegen. Bei dieser durch das Befestigen des Austragsrohrs (80) verursachten Zwangsbewegung des verschiebbaren Verschlusses (60, 260) werden die Öffnungen (40, 240) der Kartuschen (30, 230) synchron freigelegt. Dabei befinden sich Perforationen bzw. Öffnungen (nicht gezeigt) der Verlängerung (86) über den Öffnungen (40, 240) der Kartuschen (30, 230). Dadurch sind die Innenräume der Kartuschen (30, 230) mit dem Innenraum der als perforierten Hohlzylinder oder als perforierten unregelmäßig oder regelmäßig geformten Hohlkörper ausgebildeten Verlängerung (86) verbunden. Bei Bewegung der Förderkolben (62, 162, 262) in Richtung Kartuschensystemkopf (50, 250) können in den Kartuschen (30, 230) enthaltene Pasten durch die freigelegten Öffnungen (40, 240) in den Innenraum Verlängerung (86) treten und sich in Richtung Auslassöffnung (81) des Austragsrohrs (80) durch den statischen Mischer (88) bewegen.

Erfindungsgemäß ist, dass der verschiebbare Verschluss (60, 260) bevorzugt als Hohlzylinder ausgebildet ist, wobei besonders bevorzugt ist, dass der Hohlzylinder nur an der dem Kartuschenkopf (31, 231) zugewandten Seite über den gesamten Querschnitt verschlossen ist.

Durch den so ausgebildeten Abschluss des Verschlusses (60, 260), der zum Beispiel eine Scheibe sein kann, wird verhindert, dass beim Austreiben der Pasten sich diese im Mischraum (20, 220) in Richtung Kartuschenboden (33) ausbreiten können.

Durch die Ausbildung als einseitig geschlossener Hohlzylinder mit in Richtung Kartuschenboden (33) offener Seite, kann für das erste Ausführungsbeispiel die Stange (70) während der Bewegung der Förderkolben (62) in Richtung Kartuschenkopf (31) einen maximalen Weg zurück legen. Dazu ist der Durchmesser der Spitze der Stange (70) inklusive der daran befestigten Rastmittel (71) kleiner als der Innendurchmesser des als Hohlzylinder ausgebildeten verschiebbaren Verschlusses (60).

Es ist auch möglich, den verschiebbaren Verschluss (60, 260) als hohlen im Querschnitt ovalen oder im Querschnitt regelmäßig oder unregelmäßig mehreckigen Körper auszubilden. Möglich ist es auch den verschiebbaren Verschluss (60, 260) als massiven Körper oder als ein aus mehreren Einzelteilen zusammensetzbaren Körper auszubilden. Der verschiebbare Verschluss (60, 260) kann aus einem oder mehreren Werkstoffen gefertigt sein, je nach Anforderungen hinsichtlich der chemischen Stabilität gegenüber den Pasten und nach den gewünschten Gleiteigenschaften.

Erfindungsgemäß können an der Innenwand (21) des Mischraums (20, 220) Zapfen, Stege oder ein Ring als Haltemittel (74) angeordnet sein. Sie begrenzen die maximale axiale Verschiebung des verschiebbaren Verschlusses (60, 260) in Richtung des Kartuschenbodens (33) oder in Richtung des Auslasses (22).

Erfindungsgemäß ist, dass der als Verlängerung dienende perforierte Hohlkörper (86) in Längsrichtung der Kartuschen (30, 230) eine Länge größer oder zumindest gleich der Länge der Öffnungen (40, 240) der Kartuschen (30, 230) hat. Dadurch wird gewährleistet, dass die gesamten Öffnungen (40, 240) der Kartuschen (30, 230) beim Befestigen des Austragsrohrs (80) freigelegt werden können. So ist eine Freilegung der Öffnungen (40, 240) über deren gesamten Querschnitt möglich.

Weiterhin ist erfindungsgemäß, dass die Haltemittel (74) in Längsrichtung der Kartuschen (30, 230) einen Abstand zu den Öffnungen (40, 240) der Kartuschen (30, 230) von mindestens der Länge der Verlängerung (86) bzw. des Verschlusses (60, 260) haben. Die Haltemittel (74) sind notwendig, damit beim Austreiben der Pasten der verschiebbare Verschluss (60, 260) nicht weiter bewegt werden kann, als es zuvor bei der Öffnung der Kartuschen (30, 230) notwendig war. Das bedeutet, mit den Haltemitteln (74, 274) soll auch die Bildung eines nicht nutzbaren Totvolumens verhindert werden.

Die Kartuschen (30, 230) umfassen einen oder mehrere Schlitze (75, 175, 275) vom Kartuschenboden (33) wenigstens bis zur Hälfte der Kartuschenlänge, die parallel zur Kartuschenachse ausgerichtet sind. Gegebenenfalls umfassen auch die Mischraumwände (21) in Längsrichtung der Kartuschen (30, 230) ausgerichtete Schlitze (75, 175, 275), die eine gleiche Länge wie die Schlitze (75, 175, 275) der Kartuschen (30, 230) haben, wobei die Anzahl der Schlitze (75, 175, 275) in den Mischraumwänden (21) gleich der Anzahl der Schlitze (75, 175, 275) in den Kartuschen (30, 230) ist und die Schlitze (75, 175, 275) der Mischraumwände (21) sich über den Schlitzen (75, 175, 275) der Kartuschen (30, 230) befinden, so dass die Innenräume der Kartuschen (30, 230) mit dem Mischraum (20, 220) miteinander verbunden sind.

Es ist erfindungsgemäß, dass die Stege (65, 165, 265) einen Querschnitt kleiner den Schlitzen (75, 175, 275) haben. Das beutet, dass beim Auspressen der Kartuschen (30, 230, 330) die Stege (65, 165, 265) durch die Schlitze (75, 175, 275) in den Kartuschen (30, 230) in Richtung Kartuschensystemkopf (50, 250) bewegt werden können. Die Schlitze (75, 175, 275) dienen als Führung für die Stege (65, 165, 265) und damit für die Förderkolben (62, 162, 262) bei der Auspressbewegung in Richtung Kartuschenkopf (31, 231).

Das erfindungsgemäße Kartuschensystems wird zur Verpackung, Lagerung und Applikation von pastenförmigen Knochenzementen, dentalen Mehrkomponentenzubereitungen, Klebstoffen, Dichtstoffen, Kosmetika und Nahrungsmitteln verwendet. Besonders geeignet ist das Mehrkomponentenkartuschensystem für die Lagerung und Applikation von pastenförmige Polymethylmethacrylat-Knochenzementen.

### Bezugszeichenliste

- 1: Kartuschensystem
- 20, 220: Mischraum
- 21: Mischraumwand
- 22, 222: Auslassöffnung
- 30, 230: Kartusche
- 31, 231: Kartuschenkopf
- 32, 132, 232: Kartuschenwand
- 33: Kartuschenboden
- 40, 240: Öffnung
- 50, 250: Kartuschensystemkopf
- 51, 251: Befestigungsmittel am Kartuschensystemkopf
- 60, 260: Verschluss
- 62, 162, 262: Förderkolben
- 65, 165, 265: Steg
- 70, 270: Stange
- 71: Rastmittel
- 72: Gegenrastmittel
- 74: Anschlag
- 75, 175, 275: Schlitz
- 76, 276: Schlitzende
- 80: Austragsrohr
- 81: Austragsrohrspitze
- 86: Verlängerung
- 88: Mischer
- 179: Ummantelung
- 290: Zentriervorrichtung
- 291: Entarretiervorrichtung
- 293: Arretierungshaken
- 294: Arretierungsstufe

## Patentansprüche

1. Kartuschensystem zum Mischen und Applizieren eines Mischguts, insbesondere eines medizinischen Zements, umfassend zumindest zwei parallel zueinander angeordnete Kartuschen (30, 230, 330) und eine Auslassöffnung (22, 222), wobei die Kartuschen (30, 230, 330) seitlich durch Kartuschenwände (32, 132, 232, 332) begrenzt sind und jeweils einen Förderkolben (62, 162, 262, 362) zum Austreiben von Ausgangskomponenten des Mischguts aus den Kartuschen (30, 230, 330) umfassen, wobei zumindest zwei Förderkolben (62, 162, 262, 362) fest miteinander verbunden sind, wobei die zumindest zwei Förderkolben (62, 162, 262, 362) über zumindest einen Steg (65, 165, 265, 365) fest miteinander verbunden sind, die Kartuschenwände (32, 132, 232, 332) der durch den Steg oder die Stege (65, 165, 265, 365) verbundenen Kartuschen (30, 230, 330) Schlitze (75, 175, 275, 375) umfassen, die parallel zur Kartuschenachse angeordnet sind, wobei die Breite der Schlitze (75, 175, 275, 375) zur Aufnahme des oder der Stege (65, 165, 265, 265) geeignet sind und bei einer Bewegung der Förderkolben (62, 162, 262, 362) in den Kartuschen (30, 230, 330) sich der zumindest eine Steg (65, 165, 265, 365) durch Bereiche der Schlitze (75, 175, 275, 375) bewegt, **dadurch gekennzeichnet, dass** ein Mischraum (20, 220) parallel zwischen den Kartuschen (30, 230, 330) angeordnet ist.

2. Kartuschensystem nach Anspruch 1, **dadurch gekennzeichnet, dass**
die Länge der Schlitze (75, 175, 275, 375) beginnend im Bereich des Kartuschenbodens (33) bis mindestens zur Hälfte der Kartuschenlänge (76, 276, 376) reicht.

3. Kartuschensystem nach Anspruch 1 oder 2, **dadurch gekennzeichnet, dass** die Kartuschenwände (32, 132, 232, 332) am Kartuschenboden (33) durch die Schlitze (75, 175, 275, 375) vollständig geschlitzt sind.

4. Kartuschensystem nach einem der vorangehenden Ansprüche, **dadurch gekennzeichnet, dass**
ein Mischraum (20, 220) derart zwischen den Kartuschen (30, 230, 330) angeordnet ist, dass die Kartuschenwände (32, 132, 232, 332) bereichsweise mit Mischraumwänden (21) des Mischraums (20, 220) in einem ausgeführt sind, wobei im Bereich der Schlitze (75, 175, 275, 375) auch die Mischraumwände (21) geschlitzt sind.

5. Kartuschensystem nach einem der vorangehenden Ansprüche, **dadurch gekennzeichnet, dass**
im Inneren des Mischraums (20, 220) eine bewegliche Stange (70) parallel zu den Förderkolben (62, 162, 262, 362) angeordnet ist, die mit den Förderkolben (62, 162, 262, 362) über zumindest einen Steg (65, 165, 265, 365) fest verbunden ist, wobei die Stange (70) an der der Auslassöffnung (22, 222) zugewandten Seite ein Rastmittel (71) umfasst und im Mischraum (20, 220) ein Gegenrastmittel (72) angebracht ist, das mit dem Rastmittel (71) der Stange (70) derart zusammenwirkt, dass eine Bewegung der Stange (70) aus dem Mischraum (20, 220) heraus und damit der Förderkolben (62, 162, 262, 362) aus den Kartuschen (30, 230, 330) heraus deutlich erschwert, insbesondere verhindert ist.

6. Kartuschensystem nach einem der vorangehenden Ansprüche, **dadurch gekennzeichnet, dass**
die Kartuschen (30, 230, 330) jeweils zumindest eine Öffnung (40, 240, 340) in den Kartuschenwänden (32, 132, 232, 332) umfassen, die die Kartuschen (30, 230, 330) mit dem Mischraum (20, 220) verbinden, dass im Mischraum (20, 220) oder am Kartuschenkopf (31, 231, 331) ein im Mischraum (20, 220) verschiebbar angeordneter Verschluss (60, 260) oder ein am Kartuschenkopf (31, 231, 331) drehbar gelagerter Verschluss derart angeordnet ist, dass er die Öffnungen (40, 240, 340) der Kartuschen (30, 230, 330) in einer Ausgangsposition verschließt und dass die Öffnungen (40, 240, 340) in einer Endposition des Verschlusses (60, 260) zumindest bereichsweise geöffnet sind, wobei der Verschluss (60, 260) von der Ausgangsposition zur Endposition verschiebbar oder drehbar ist.

7. Kartuschensystem nach Anspruch 6, **dadurch gekennzeichnet, dass** im Mischraum (20, 220) ein Anschlag (74), vorzugsweise in Form von Zapfen oder Stegen, angeordnet ist, der die Bewegung des verschiebbaren Verschlusses (60, 260) im Mischraum (20, 220) begrenzt und dadurch die Endposition des verschiebbaren Verschlusses (60, 260) definiert.

8. Kartuschensystem nach einem der vorangehenden Ansprüche, **dadurch gekennzeichnet, dass**
die Auslassöffnung (22, 222) in einem Kartuschensystemkopf (50, 250) angeordnet ist, und der Kartuschensystemkopf (50, 250) ein Befestigungsmittel (51, 251) zum Befestigen eines Austragsrohrs (80, 380) umfasst, insbesondere auf der Innenseite des Kartuschensystemkopfs (50, 250), vorzugsweise ein Gewinde oder mehrere Zapfen.

9. Kartuschensystem nach einem der vorangehenden Ansprüche, **dadurch gekennzeichnet, dass**
die Förderkolben (62, 162, 262, 362) die Kartuschen (30, 230, 330) dicht, insbesondere gasdicht, verschließen, vorzugsweise oberhalb des Schlitzendes (76, 276, 376).

10. Kartuschensystem nach einem der vorangehenden Ansprüche, **dadurch gekennzeichnet, dass**
der Steg (65, 165, 265, 365) oder zumindest einer der Stege (65, 165, 265, 365) bodenseitig mit den Förderkolben (62, 162, 262, 362) fest verbunden ist, und ein Arretierungsmittel (70, 71, 293) mit dem zumindest einen Steg (65, 165, 265, 365) verbunden ist, wobei das Arretierungsmittel (70, 71, 293) einen Bereich (20, 220) parallel oder zwischen den Kartuschen (30, 230, 330) reicht und dort in ein Gegenarretierungsmittel (72, 294) greift, so dass eine Bewegung des zumindest einen Stegs (65, 165, 265, 365) und der Förderkolben (62, 162, 262, 362) in Richtung des Kartuschenbodens (33) blockiert ist.

11. Kartuschensystem nach Anspruch 10, **dadurch gekennzeichnet, dass** in dem Bereich (20, 220) eine Entarretiervorrichtung (291) vorgesehen ist, die fest mit dem verschiebbaren Verschluss (60, 260) verbunden ist, so dass beim Verschieben des verschiebbaren Verschlusses (60, 260) in seine Endpostition, eine Entarretierung der Arretierungsmittel (70, 71, 293) erfolgt, so dass eine Bewegung des zumindest einen Stegs (65, 165, 265, 365) und der Förderkolben (62, 162, 262, 362) in Richtung des Kartuschenbodens (33) möglich ist.

12. Kartuschensystem nach einem der vorangehenden Ansprüche, **dadurch gekennzeichnet, dass**
die Förderkolben (62, 162, 262, 362) und die Kartuschenwände (32, 132, 232, 332) gasdicht und miteinander schlüssig aufgebaut sind, so dass durch einen Gasdruck auf den Kartuschenboden (33) die Förderkolben (62, 162, 262, 362) in die Kartuschen (30, 230, 330) drückbar sind.

## Claims

1. Cartridge system for mixing and applying mixing goods, in particular a medical cement, comprising at least two cartridges (30, 230, 330) that are arranged to be parallel to each other and an outlet opening (22, 222), whereby the cartridges (30, 230, 330) are bordered on the side by cartridge walls (32, 132, 232, 332) and each comprise a feed plunger (62, 162, 262, 362) for expelling starting components of the mixing goods from the cartridges (30, 230, 330), whereby at least two feed plungers (62, 162, 262, 362) are firmly connected to each other, whereby the at least two feed plungers (62, 162, 262, 362) are firmly connected to each other by means of at least one fin (65, 165, 265, 365), [and] the cartridge walls (32, 132, 232, 332) of the cartridges (30, 230, 330) connected by means of the fin or fins (65, 165, 265, 365) comprise slits (75, 175, 275, 375) that are arranged to be parallel to the cartridge axis, whereby the width of the slits (75, 175, 275, 375) is suitable for accommodating the fin or fins (65, 165, 265, 265) and, upon a motion of the feed plungers (62, 162, 262, 362) in the cartridges (30, 230, 330), the at least one fin (65, 165, 265, 365) moves through regions of the slits (75, 175, 275, 375), **characterised in that** a mixing space (20, 220) is arranged to be parallel between the cartridges (30, 230, 330).

2. Cartridge system according to claim 1, **characterised in that**
the length of the slits (75, 175, 275, 375) extends from the region of the cartridge bottom (33) to at least half of the cartridge length (76, 276, 376).

3. Cartridge system according to claim 1 or 2, **characterised in that**
the cartridge walls (32, 132, 232, 332) on the cartridge bottom (33) are fully slit by the slits (75, 175, 275, 375).

4. Cartridge system according to any one of the preceding claims, **characterised in that**
a mixing space (20, 220) is arranged appropriately between the cartridges (30, 230, 330) such that regions of the cartridge walls (32, 132, 232, 332) are provided to also serve as mixing space walls (21) of the mixing space (20, 220), whereby the mixing space walls (21) are also slit in the region of the slits (75, 175, 275, 375).

5. Cartridge system according to any one of the preceding claims, **characterised in that**
a mobile rod (70) is arranged appropriately on the inside of the mixing space (20, 220) such as to be parallel to the feed plungers (62, 162, 262, 362) and is firmly connected to the feed plungers (62, 162, 262, 362) by means of at least one fin (65, 165, 265, 365), whereby the rod (70) comprises a snap-in means (71) on the side facing the outlet opening (22, 222) and an opposite snap-in means (72) is attached in the mixing space (20, 220) and acts in concert appropriately with the snap-in means (71) of the rod (70) such that a motion of the rod (70) out of the mixing space (20, 220) and thus of the feed plungers (62, 162, 262, 362) out of the cartridges (30, 230, 330) is made significantly more difficult, in particular is prevented.

6. Cartridge system according to any one of the preceding claims, **characterised in that**
the cartridges (30, 230, 330) each comprise at least one opening (40, 240, 340) in the cartridge walls (32, 132, 232, 332) that connect the cartridges (30, 230, 330) to the mixing space (20, 220), **in that** a closure (60, 260) that is arranged such that it can be shifted in the mixing space (20, 220) or a closure that is supported as in a bearing on the cartridge head (31, 231, 331) such that it can rotate is arranged in the mixing space (20, 220) or on the cartridge head (31, 231, 331) such that the closure closes the openings (40, 240, 340) of the cartridges (30, 230, 330) in a starting position, and **in that** the openings (40, 240, 340) are open, at least regions thereof, in an end position of the closure (60, 260), whereby the closure (60, 260) can be shifted or rotated from the starting position to the end position.

7. Cartridge system according to claim 6, **characterised in that**
a limit stop (74), preferably in the form of pegs or fins, is arranged in the mixing space (20, 220) and limits the motion of the shiftable closure (60, 260) in the mixing space (20, 220) and thus defines the end position of the shiftable closure (60, 260).

8. Cartridge system according to any one of the preceding claims, **characterised in that**
the outlet opening (22, 222) is arranged in a cartridge system head (50, 250), and the cartridge system head (50, 250) comprises a fastening means (51, 251) for fastening a dispensing tube (80, 380), in particular on the inside of the cartridge system head (50, 250), preferably a thread or multiple pegs.

9. Cartridge system according to any one of the preceding claims, **characterised in that**
the feed plungers (62, 162, 262, 362) close the cartridges (30, 230, 330) in tight manner, in particular in gas-tight manner, preferably above the slit end (76, 276, 376).

10. Cartridge system according to any one of the preceding claims, **characterised in that**
the fin (65, 165, 265, 365) or at least one of the fins (65, 165, 265, 365) is firmly connected, on the bottom-side, to the feed plungers (62, 162, 262, 362), and a locking means (70, 71, 293) is connected to the at least one fin (65, 165, 265, 365), whereby the locking means (70, 71, 293) extends into a region (20, 220) parallel to or between the cartridges (30, 230, 330) and there engages an opposite locking means (72, 294) such that a motion of the at least one fin (65, 165, 265, 365) and of the feed plungers (62, 162, 262, 362) in the direction of the cartridge bottom (33) is blocked.

11. Cartridge system according to claim 10, **characterised in that**
an unlocking device (291) that is firmly connected to the shiftable closure (60, 260) is provided in the region (20, 220), such that, upon shifting the shiftable closure (60, 260) into its final position, the locking means (70, 71, 293) become unlocked, such that a motion of the at least one fin (65, 165, 265, 365) and of the feed plungers (62, 162, 262, 362) in the direction of the cartridge bottom (33) is feasible.

12. Cartridge system according to any one of the preceding claims, **characterised in that**
the feed plungers (62, 162, 262, 362) and the cartridge walls (32, 132, 232, 332) are designed to be gas-tight and flush with respect to each other, such that the feed plungers (62, 162, 262, 362) can be pushed into the cartridges (30, 230, 330) by means of a gas pressure acting on the cartridge bottom (33).

## Revendications

1. Système de cartouches pour mélanger et appliquer une marchandise mélangée, en particulier un ciment médical, comprenant au moins deux cartouches (30, 230, 330) disposées parallèles entre elles et une ouverture d'évacuation (22, 222), sachant que les cartouches (30, 230, 330) sont délimitées latéralement par des parois de cartouches (32, 132, 232, 332) et comprennent respectivement un piston d'alimentation (62, 162, 262, 362) pour faire sortir des composantes de départ de la marchandise mélangée hors des cartouches (30, 230, 330), sachant qu'au moins deux pistons d'alimentation (62, 162, 262, 362) sont reliés fixement entre eux, sachant que les au moins deux pistons d'alimentation (62, 162, 262, 362) sont reliés fixement entre eux par une barre (65, 165, 265, 365), les parois de cartouches (32, 132, 232, 332) des cartouches (30, 230, 330) reliées par la/les barre(s) (65, 165, 265, 365) comprennent des fentes (75, 175, 275, 375) qui sont disposées parallèlement à l'axe des cartouches, sachant que la largeur des fentes (75, 175, 275, 375) est appropriée pour recevoir la/les barre(s) (65, 165, 265, 365), et lors d'un mouvement des pistons d'alimentation (62, 162, 262, 362) dans les cartouches (30, 230, 330), l'au moins une barre (65, 165, 265, 365) se déplace à travers des tronçons des fentes (75, 175, 275, 375), **caractérisé en ce qu'**un espace de mélange (20, 220) est disposé parallèlement entre les cartouches (30, 230, 330).

2. Système de cartouches selon la revendication 1, **caractérisé en ce que** la longueur des fentes (75, 175, 275, 375) part du niveau du fond de cartouche (33) jusqu'au moins la moitié de la longueur de cartouche (76, 276, 376).

3. Système de cartouches selon la revendication 1 ou 2, **caractérisé en ce que** les parois de cartouches (32, 132, 232, 332) sont totalement fendues par les fentes (75, 175, 275, 375) sur le fond de cartouche (33).

4. Système de cartouches selon l'une quelconque des revendications précédentes, **caractérisé en ce qu'**un espace de mélange (20, 220) est disposé de telle façon entre les cartouches (30, 230, 330), que les parois de cartouches (32, 132, 232, 332) sont conçues par tronçons comme un tout avec des parois d'espace de mélange (21) de l'espace de mélange (20, 220), sachant qu'au niveau des fentes (75, 175, 275, 375), les parois d'espace de mélange (21) sont également fendues.

5. Système de cartouches selon l'une quelconque des revendications précédentes, **caractérisé en ce qu'**une barre mobile (70) parallèle aux pistons d'alimentation (62, 162, 262, 362) est disposée à l'intérieur de l'espace de mélange (20, 220), laquelle est reliée fixement aux pistons d'alimentation (62, 162, 262, 362) par le biais d'au moins une barre (65, 165, 265, 365), sachant que la barre (70) comprend un moyen d'encliquetage (71) sur le côté tourné vers l'ouverture d'évacuation (22, 222), et un moyen de contre-encliquetage (72) est disposé dans l'espace de mélange (20, 220), lequel coopère avec le moyen d'encliquetage (71) de la barre (70) de telle façon qu'un mouvement de la barre (70) hors de l'espace de mélange (20, 220) et donc des pistons d'alimentation (62, 162, 262, 362) hors des cartouches (30, 230, 330) est significativement compliqué, est empêché en particulier.

6. Système de cartouches selon l'une quelconque des revendications précédentes, **caractérisé en ce que** les cartouches (30, 230, 330) comprennent respectivement au moins une ouverture (40, 240, 340) dans les parois de cartouches (32, 132, 232, 332) qui relient les cartouches (30, 230, 330) à l'espace de mélange (20, 220), qu'une fermeture (60, 260) disposée mobile dans l'espace de mélange (20, 220) ou qu'une fermeture positionnée rotative sur la tête de cartouche (31, 231, 331) est prévue dans l'espace de mélange (20, 220) ou sur la tête de cartouche (31, 231, 331), est disposée de telle façon qu'elle ferme les ouvertures (40, 240, 340) des cartouches (30, 230, 330) dans une position de départ et que les ouvertures (40, 240, 340) sont ouvertes au moins par tronçons dans une position de fin de la fermeture (60, 260), sachant que la fermeture (60, 260) est mobile ou rotative de la position de départ à la position de fin.

7. Système de cartouches selon la revendication 6, **caractérisé en ce qu'**une butée (74), de préférence sous forme de taquet ou de barre, est disposée dans l'espace de mélange (20, 220), qui délimite le mouvement de la fermeture mobile (60, 260) dans l'espace de mélange (20, 220) et définit ainsi la position de fin de la fermeture mobile (60, 260).

8. Système de cartouches selon l'une quelconque des revendications précédentes, **caractérisé en ce que** l'ouverture d'évacuation (22, 222) est disposée dans une tête de système de cartouche (50, 250) et la tête de système de cartouche (50, 250) comprend un moyen de fixation (51, 251) pour fixer un tube distributeur (80, 380), en particulier sur la face intérieure de la tête de système de cartouche (50, 250), de préférence un filetage ou plusieurs taquets.

9. Système de cartouches selon l'une quelconque des revendications précédentes, **caractérisé en ce que** les pistons d'alimentation (62, 162, 262, 362) ferment les cartouches (30, 230, 330) de façon étanche, en particulier étanche aux gaz, de préférence au-dessus de l'extrémité de la fente (76, 276, 376).

10. Système de cartouches selon l'une quelconque des revendications précédentes, **caractérisé en ce que** la barre (65, 165, 265, 365) ou au moins une des barres (65, 165, 265, 365) est reliée fixement aux pistons d'alimentation (62, 162, 262, 362) par le fond, et un moyen de blocage (70, 71, 293) est relié à au moins une barre (65, 165, 265, 365), sachant que le moyen de blocage (70, 71, 293) atteint une partie (20, 220) parallèle aux ou entre les cartouches (30, 230, 330) et se met en prise à cet endroit dans un moyen de contre-blocage (72, 294), de telle sorte qu'un mouvement de l'au moins une barre (65, 165, 265, 365) et des pistons d'alimentation (62, 162, 262, 362) en direction du fond de cartouche (33) est bloqué.

11. Système de cartouches selon la revendication 10, **caractérisé en ce que** dans la partie (20, 220) est prévu un dispositif de déblocage (291) qui est relié fixement à la fermeture mobile (60, 260), de sorte que lorsque la fermeture mobile (60, 260) est déplacée dans sa position de fin, un déblocage du moyen de blocage (70, 71, 293) a lieu, de sorte qu'un mouvement de l'au moins une barre (65, 165, 265, 365) et des pistons d'alimentation (62, 162, 262, 362) en direction du fond de cartouche (33) est possible.

12. Système de cartouches selon l'une quelconque des revendications précédentes, **caractérisé en ce que** les pistons d'alimentation (62, 162, 262, 362) et les parois de cartouches (32, 132, 232, 332) sont montés par complémentarité de forme entre eux et étanches aux gaz, de telle sorte que les pistons d'alimentation (62, 162, 262, 362) peuvent être comprimés dans les cartouches (30, 230, 330) par une pression gazeuse sur le fond de cartouche (33).
